# EUROPEAN PATENT SPECIFICATION

(11) **EP 2 288 732 B1**
(45) Date of publication and mention of the grant of the patent: **02.10.2013**
(21) Application number: 09761778.1
(22) Date of filing: 12.06.2009
(51) Int. Cl.: C12Q 1/68

(54) **A METHOD FOR PREDICTING THE RESPONSE TO A TREATMENT WITH ANAKINRA**
VERFAHREN ZUR VORHERSAGE DES ANSPRECHENS AUF EINE BEHANDLUNG MIT ANAKINRA
PROCÉDÉ POUR PRÉVOIR LA RÉPONSE À UN TRAITEMENT À L'ANAKINRA

(30) Priority: 12.06.2008 EP 08305252; 11.05.2009 US 176979 P
(43) Date of publication of application: 02.03.2011
(73) Proprietor: INSERM (Institut National de la Santé et de la Recherche Médicale), 75013 Paris (FR)
(72) Inventor: LEQUERRE, Thierry, F-76183 Rouen Cedex (FR); LE LOËT, Xavier, 76183 Rouen Cedex (FR); VITTECOQ, Olivier, 76183 Rouen Cedex (FR); SALIER, Jean-Philippe, 76183 Rouen Cedex (FR)
(74) Representative: Regimbeau
(86) International application number: PCT/EP2009/057267
(87) International publication number: WO 2009/150216

(56) References cited:
- EP-A- 1 857 559
- WO-A-2004/107240
- BANSARD ET AL: "Identification de marqueurs predictifs de reponse a l'anakinra chez des patients atteints de polyarthrite rhumatoide" REVUE DU RHUMATISME, EDITIONS SCIENTIFIQUES ET MEDICALES ELSEVIER, vol. 74, no. 10-11, 1 November 2007 (2007-11-01), pages 1021-1022, XP022488073 ISSN: 1169-8330
- 19000101, 1 January 1900 (1900-01-01), XP008096364
- LEQUERRÉ THIERRY ET AL: "Gene profiling in white blood cells predicts infliximab responsiveness in rheumatoid arthritis" ARTHRITIS RESEARCH AND THERAPY, BIOMED CENTRAL, LONDON, GB, vol. 8, no. 4, 3 July 2006 (2006-07-03), page R105, XP021020585 ISSN: 1478-6354 cited in the application
- LEQUERRE ET AL: "What is the role for interleukin-1 receptor antagonist in rheumatic disease?" JOINT BONE SPINE, ELSEVIER, PARIS, FR, vol. 74, no. 3, 1 May 2007 (2007-05-01), pages 223-226, XP022071757 ISSN: 1297-319X
- DATABASE MEDLINE [Online] US NATIONAL LIBRARY OF MEDICINE (NLM), BETHESDA, MD, US October 2010 JUNG NORMA ET AL: 'An open-label pilot study of the efficacy and safety of anakinra in patients with psoriatic arthritis refractory to or intolerant of methotrexate (MTX).' Database accession no. NLM20532937 & JUNG NORMA ET AL: "An open-label pilot study of the efficacy and safety of anakinra in patients with psoriatic arthritis refractory to or intolerant of methotrexate (MTX).", CLINICAL RHEUMATOLOGY OCT 2010, vol. 29, no. 10, October 2010 (2010-10), pages 1169-1173, ISSN: 1434-9949
- DATABASE MEDLINE [Online] US NATIONAL LIBRARY OF MEDICINE (NLM), BETHESDA, MD, US September 1996 CARON J P ET AL: 'Chondroprotective effect of intraarticular injections of interleukin-1 receptor antagonist in experimental osteoarthritis. Suppression of collagenase-1 expression.' Database accession no. NLM8814066 & CARON J P ET AL: "Chondroprotective effect of intraarticular injections of interleukin-1 receptor antagonist in experimental osteoarthritis. Suppression of collagenase-1 expression.", ARTHRITIS AND RHEUMATISM SEP 1996, vol. 39, no. 9, September 1996 (1996-09), pages 1535-1544, ISSN: 0004-3591
- DATABASE MEDLINE [Online] US NATIONAL LIBRARY OF MEDICINE (NLM), BETHESDA, MD, US October 1994 SIM T C ET AL: 'Interleukin-1 receptor antagonist protein inhibits the synthesis of IgE and proinflammatory cytokines by allergen-stimulated mononuclear cells.' Database accession no. NLM7917315 & SIM T C ET AL: "Interleukin-1 receptor antagonist protein inhibits the synthesis of IgE and proinflammatory cytokines by allergen-stimulated mononuclear cells.", AMERICAN JOURNAL OF RESPIRATORY CELL AND MOLECULAR BIOLOGY OCT 1994, vol. 11, no. 4, October 1994 (1994-10), pages 473-479, ISSN: 1044-1549
- DATABASE EMBASE [Online] ELSEVIER SCIENCE PUBLISHERS, AMSTERDAM, NL 1995 MARTIN D ET AL: 'Protective effect of the interleukin-1 receptor antagonist (IL-1ra) on experimental allergic encephalomyelitis in rats' Database accession no. EMB-1995313419 & MARTIN D ET AL: "Protective effect of the interleukin-1 receptor antagonist (IL-1ra) on experimental allergic encephalomyelitis in rats", JOURNAL OF NEUROIMMUNOLOGY 1995 NL, vol. 61, no. 2, 1995, pages 241-245, ISSN: 0165-5728
- DATABASE MEDLINE [Online] US NATIONAL LIBRARY OF MEDICINE (NLM), BETHESDA, MD, US November 2011 SUMPTER KATHRYN M ET AL: 'Preliminary studies related to anti-interleukin-1[beta] therapy in children with newly diagnosed type 1 diabetes.' Database accession no. NLM21518168 & SUMPTER KATHRYN M ET AL: "Preliminary studies related to anti-interleukin-1[beta] therapy in children with newly diagnosed type 1 diabetes.", PEDIATRIC DIABETES NOV 2011, vol. 12, no. 7, November 2011 (2011-11), pages 656-667, ISSN: 1399-5448
- DATABASE EMBASE [Online] ELSEVIER SCIENCE PUBLISHERS, AMSTERDAM, NL 1994 SANDBERG J -O ET AL: 'Interleukin-1 receptor antagonist prevents low dose streptozotocin induced diabetes in mice' Database accession no. EMB-1994245779 & SANDBERG J -O ET AL: "Interleukin-1 receptor antagonist prevents low dose streptozotocin induced diabetes in mice", BIOCHEMICAL AND BIOPHYSICAL RESEARCH COMMUNICATIONS 1994 US, vol. 202, no. 1, 1994, pages 543-548, ISSN: 0006-291X
- TAN A L ET AL: "Efficacy of anakinra in active ankylosing spondylitis: a clinical and magnetic resonance imaging study.", ANNALS OF THE RHEUMATIC DISEASES SEP 2004, vol. 63, no. 9, September 2004 (2004-09), pages 1041-1045, ISSN: 0003-4967
- SO ALEXANDER ET AL: "A pilot study of IL-1 inhibition by anakinra in acute gout.", ARTHRITIS RESEARCH & THERAPY 2007, vol. 9, no. 2, 2007, page R28, ISSN: 1478-6362
- SANDBERG J O ET AL: "IL-1 receptor antagonist inhibits recurrence of disease after syngeneic pancreatic islet transplantation to spontaneously diabetic non-obese diabetic (NOD) mice.", CLINICAL AND EXPERIMENTAL IMMUNOLOGY MAY 1997, vol. 108, no. 2, May 1997 (1997-05), pages 314-317, ISSN: 0009-9104
- PFLEGER CHRISTIAN ET AL: "Association of IL-1ra and adiponectin with C-peptide and remission in patients with type 1 diabetes.", DIABETES APR 2008, vol. 57, no. 4, April 2008 (2008-04), pages 929-937, ISSN: 1939-327X
- TEOH S C B ET AL: "Tailoring biological treatment: anakinra treatment of posterior uveitis associated with the CINCA syndrome.", THE BRITISH JOURNAL OF OPHTHALMOLOGY FEB 2007, vol. 91, no. 2, February 2007 (2007-02), pages 263-264, ISSN: 0007-1161
- LIM WEE-KIAK ET AL: "Suppression of immune-mediated ocular inflammation in mice by interleukin 1 receptor antagonist administration", ARCHIVES OF OPHTHALMOLOGY, vol. 123, no. 7, July 2005 (2005-07), pages 957-963, ISSN: 0003-9950
- HAWKINS PHILIP N ET AL: "Spectrum of clinical features in Muckle-Wells syndrome and response to anakinra.", ARTHRITIS AND RHEUMATISM FEB 2004, vol. 50, no. 2, February 2004 (2004-02), pages 607-612, ISSN: 0004-3591
- LESLIE KIERON S ET AL: "Phenotype, genotype, and sustained response to anakinra in 22 patients with autoinflammatory disease associated with CIAS-1/NALP3 mutations.", ARCHIVES OF DERMATOLOGY DEC 2006, vol. 142, no. 12, December 2006 (2006-12), pages 1591-1597, ISSN: 0003-987X

### SEQUENCE LISTING

<110> INSERM
<120> A METHOD FOR PREDICTING THE RESPONSE TO A TREATMENT WITH ANAKINRA
<130> BIO08266 LEQUERRE / MC
<160> 42
<170> PatentIn version 3.3
<210> 1
   <211> 20
   <212> DNA
   <213> Artificial
<220>
   <223> Primer
<400> 1
   cgcggggctt aatacatagg 20
<210> 2
   <211> 20
   <212> DNA
   <213> Artificial
<220>
   <223> primer
<400> 2
   gtacctgctc ggcatggagt 20
<210> 3
   <211> 20
   <212> DNA
   <213> Artificial
<220>
   <223> Primer
<400> 3
   ggcagacgca gcttagagag 20
<210> 4
   <211> 20
   <212> DNA
   <213> Artificial
<220>
   <223> Primer
<400> 4
   ccgctctctt gctttatcca 20
<210> 5
   <211> 20
   <212> DNA
   <213> Artificial
<220>
   <223> Primer
<400> 5
   catggtgact ggaaacatgc 20
<210> 6
   <211> 20
   <212> DNA
   <213> Artificial
<220>
   <223> Primer
<400> 6
   gatctgcact tggacagcaa 20
<210> 7
   <211> 20
   <212> DNA
   <213> Artificial
<220>
   <223> Primer
<400> 7
   cccctcttct ctctgcctga 20
<210> 8
   <211> 20
   <212> DNA
   <213> Artificial
<220>
   <223> Primer
<400> 8
   ggcctgcaac ccattcttag 20
<210> 9
   <211> 20
   <212> DNA
   <213> Artificial
<220>
   <223> Primer
<400> 9
   cggattgttg tgatgactgc 20
<210> 10
   <211> 20
   <212> DNA
   <213> Artificial
<220>
   <223> Primer
<400> 10
   tgtcatttcc ttctgcctga 20
<210> 11
   <211> 20
   <212> DNA
   <213> Artificial
<220>
   <223> Primer
<400> 11
   cctggccctc agagattttg 20
<210> 12
   <211> 20
   <212> DNA
   <213> Artificial
<220>
   <223> Primer
<400> 12
   aaacggcaat caggtcgaat 20
<210> 13
   <211> 20
   <212> DNA
   <213> Artificial
<220>
   <223> Primer
<400> 13
   acaggaggag aaacccagca 20
<210> 14
   <211> 20
   <212> DNA
   <213> Artificial
<220>
   <223> Primer
<400> 14
   gaagaagtgg ggcatgaagc 20
<210> 15
   <211> 20
   <212> DNA
   <213> Artificial
<220>
   <223> Primer
<400> 15
   ccacctccga tcatcagtca 20
<210> 16
   <211> 20
   <212> DNA
   <213> Artificial
<220>
   <223> Primer
<400> 16
   ggtgagcagc aggtgaacag 20
<210> 17
   <211> 20
   <212> DNA
   <213> Artificial
<220>
   <223> Primer
<400> 17
   acctgatgga cctgggattg 20
<210> 18
   <211> 20
   <212> DNA
   <213> Artificial
<220>
   <223> Primer
<400> 18
   tttgtcaccc cagcgtactg 20
<210> 19
   <211> 20
   <212> DNA
   <213> Artificial
<220>
   <223> Primer
<400> 19
   tgcggagctg gtaagaatca 20
<210> 20
   <211> 20
   <212> DNA
   <213> Artificial
<220>
   <223> Primer
<400> 20
   cccaggggac tgtgaaagag 20
<210> 21
   <211> 20
   <212> DNA
   <213> Artificial
<220>
   <223> Primer
<400> 21
   ttttgtacct cggggacctg 20
<210> 22
   <211> 20
   <212> DNA
   <213> Artificial
<220>
   <223> Primer
<400> 22
   gccgtatttg ctcatccaca 20
<210> 23
   <211> 20
   <212> DNA
   <213> Artificial
<220>
   <223> Primer
<400> 23
   aacaggctcg ggttgctaaa 20
<210> 24
   <211> 20
   <212> DNA
   <213> Artificial
<220>
   <223> Primer
<400> 24
   catttgccgc ttcttccttc 20
<210> 25
   <211> 19
   <212> DNA
   <213> Artificial
<220>
   <223> Primer
<400> 25
   aaagcaggag ccggtctgt 19
<210> 26
   <211> 20
   <212> DNA
   <213> Artificial
<220>
   <223> Primer
<400> 26
   ggggtcttgt ctgccttcac 20
<210> 27
   <211> 20
   <212> DNA
   <213> Artificial
<220>
   <223> Primer
<400> 27
   gctcatcctc ctgctcatcc 20
<210> 28
   <211> 20
   <212> DNA
   <213> Artificial
<220>
   <223> Primer
<400> 28
   cgttgccgat ggtctgatta 20
<210> 29
   <211> 20
   <212> DNA
   <213> Artificial
<220>
   <223> Primer
<400> 29
   gcgcaagtcg agaggaaact 20
<210> 30
   <211> 20
   <212> DNA
   <213> Artificial
<220>
   <223> Primer
<400> 30
   actccacacg gggagaagaa 20
<210> 31
   <211> 20
   <212> DNA
   <213> Artificial
<220>
   <223> Primer
<400> 31
   gcgtgaatcc ggaagaaaag 20
<210> 32
   <211> 20
   <212> DNA
   <213> Artificial
<220>
   <223> Primer
<400> 32
   ctgttgtggt cccatctcca 20
<210> 33
   <211> 20
   <212> DNA
   <213> Artificial
<220>
   <223> Primer
<400> 33
   tgggagaaca ggcacaagaa 20
<210> 34
   <211> 20
   <212> DNA
   <213> Artificial
<220>
   <223> Primer
<400> 34
   gacaccacca ccatgaccac 20
<210> 35
   <211> 20
   <212> DNA
   <213> Artificial
<220>
   <223> Primer
<400> 35
   aatcccagca ctttcggaga 20
<210> 36
   <211> 20
   <212> DNA
   <213> Artificial
<220>
   <223> Primer
<400> 36
   aagcgattct cctgcctcag 20
<210> 37
   <211> 20
   <212> DNA
   <213> Artificial
<220>
   <223> Primer
<400> 37
   atgcctcagc ctcccaaata 20
<210> 38
   <211> 20
   <212> DNA
   <213> Artificial
<220>
   <223> Primer
<400> 38
   aggccccagg atatgctttt 20
<210> 39
   <211> 20
   <212> DNA
   <213> Artificial
<220>
   <223> Primer
<400> 39
   ctgctcagcg ttgccataag 20
<210> 40
   <211> 20
   <212> DNA
   <213> Artificial
<220>
   <223> Primer
<400> 40
   ggcttggaag gtggtgtagg 20
<210> 41
   <211> 20
   <212> DNA
   <213> Artificial
<220>
   <223> Primer
<400> 41
   gtggagcgat ttgtctggtt 20
<210> 42
   <211> 20
   <212> DNA
   <213> Artificial
<220>
   <223> Primer
<400> 42
   cgctgagcca gtcagtgtag 20

## Description

### FIELD OF THE INVENTION:

The present invention relates to a method for predicting the response of patient to a treatment with anakinra.

### BACKGROUND OF THE INVENTION:

Reumathoid arthritis (RA) is a chronic, auto-immune and inflammatory polyarthritis which induces joint damage and disability. Thanks to the better understanding of RA physiopathology, several anti-cytokines targeted against TNFα, IL-1β or IL-6 and several cellular immunotherapies (anti-CD20 or CTLA-4Ig) have been successfully introduced for RA treatment. The rational for using IL-1 receptor antagonist (IL-1Ra) to treat RA originates in studies showing high levels of IL-1β transcripts in the rheumatoid synovium, as well as high levels of IL-1β in serum and synovial fluid that correlated closely to disease activity. The IL-1 excess leads to an IL-1/IL-1Ra imbalance, which generates chronic inflammation with leukocytic infiltration, synovial hyperplasia, invasive pannus and erosion of the cartilage and bone. Since IL-1 is one of the pivotal cytokines in initiating disease and IL-1Ra is deficient in RA, recombinant IL-1Ra has been investigated in RA treatments. Several studies have shown that recombinant IL-1 Ra [anakinra (Kineret®)] improves the symptoms and signs of RA, slows bone and joint destruction, and improves quality of life, without inducing major adverse effects, even in patients with comorbid conditions. However, on clinical practice, the effects of anakinra on disease activity are weaker than those obtained with anti-TNF agents, probably because of the limited bioavailability of anakinra over the 24-h cycle. Nevertheless, anakinra combined with methotrexate is strongly effective in some patients. Indeed, in a prospective study, about 10% to 15% of patients enjoyed lasting benefits after 2 years, suggesting that a subset of patients may be particularly responsive to the anakinra/methotrexate combination. Furthermore, patients who had discontinued anti-TNF therapy because of lack efficacy and/or adverse events demonstrated improvements after 6 months of anakinra therapy with an excellent safety profile. The cytokine profile probably varies across patients and over time in the individual patient with RA, and IL-1 inhibition is probably effective only during a specific window in the pathophysiological process. In this context, anakinra in combination with methotrexate is an effective and safe treatment for a subset of RA patients whose profile remains to be defined. The latest European recommendations have also recognized the anakinra efficacy in RA but have suggested that its specific place must be defined in the rheumatological armamentarium. For these reasons, predicting responsiveness to anakinra associated with methotrexate would be of great interest and useful. Determination of cellularly produced IL-1β and even more of the IL-1Ra/IL-1β synthesis by PBMC may be useful to predict the outcome of RA patients undergoing treatment with methotrexate and may characterize a subset of RA that is more responsive to IL-1 directed therapeutic interventions. But, at the present time, any markers have been identified that might truly predict the responsiveness to anakinra.

Bansard et al. (Revue du rhumatisme, Editions scientifiques et médicales Elsevier, vol. 74, no. 10-11, 007-11-01, p. 1021-1022, SSN: 1169-8330) relates to a method for predicting the response of a patient affected with rheumatoid arthritis to a treatment with anakinra by measuring gene expression levels.

### SUMMARY OF THE INVENTION:

The present invention relates to a method for predicting the response of a patient to a treatment with anakinra, said method comprising a step of measuring the expression level of 7 genes in a biological sample of said patient, wherein said genes are GTF2F2, CCT3, CROT, HNRPA3, ARL15, TMED5, and NRG3.

The patient is affected with an IL-1 related disease selected from rheumatoid arthritis, spondyloarthropathies, osteoarthritis, gouty arthritis, allergy, multiple sclerosis, autoimmune diabetes, autoimmune uveitis, and nephrotic syndrome.

In a particular embodiment said patient is affected with rheumatoid arthritis. In another embodiment said patient is affected with rheumatoid arthritis that is active.

In another embodiment the method of the invention further comprises a step of measuring the expression level of 13 genes in said biological sample of said patient, wherein said genes are SERPINE1, MRPL40, EIF3S12, OVGP1, ZDHHC20, BAZ2B, F11R, SLC11A2, ONECUT1, MAP4, SLC15A4, CLEC2D, and RBM35B.

In another embodiment the method of the invention further comprises a step of comparing the combined expression level of said genes with reference values obtained from responder and non-responder group of patients.

In another embodiment the patient is treated with methotrexate, azathropine or lefunomide.

The present invention also relates to the use of anakinra for the preparation of a medicament for treating a patient with an IL-I related disease selected from rheumatoid arthritis, spondylarthropathies, osteoarthritis, gouty arthritis, allergy, multiple sclerosis, autoimmune diabetes, autoimmune uveitis, and nephrotic syndrome, wherein the patient has been classified as a responder to a treatment with anakinra by determining the expression level of 7 genes in a biological sample of said patient, wherein said genes are GTF2F2, CCT3, CROT, HNRPA3, ARL15, TMED5, and NRG3.

In a particular embodiment the patient is affected with rheumatoid arthritis.

### DETAILED DESCRIPTION OF THE INVENTION:

### Definitions:

The term "anakinra" refers to an interleukin-1 (IL-1) receptor antagonist. The anakinra molecule is a recombinant, non glycosolated version of human IL-1 receptor antagonist (IL-1RA) (Cohen S. et al. 2002). It consists of 153 amino acids and has a molecular weight of 17,257.6 g/mol (approx. 17.3 kDa) and differs from native human IL-1RA in that it has the addition of a single methionine residue on its amino terminus.

The term "patient" refers to any subject (preferably human) afflicted with a disease likely to benefit from a treatment with anakinra, in particular an IL-1 related disease.

The IL-1 related diseases encompass an autoimmune disorder, a spondyloarthropathy, a metabolic disorder, pain, and vasculitis. In one embodiment, the autoimmune disorder is selected from the group consisting of rheumatoid arthritis, spondylarthropathies, osteoarthritis, gouty arthritis, allergy, multiple sclerosis, autoimmune diabetes, autoimmune uveitis, and nephrotic syndrome. In another aspect, the IL-1 related disease is selected from the group consisting of inflammatory bone disorders, bone resorption disease, alcoholic hepatitis, viral hepatitis, fulminant hepatitis, coagulation disturbances, bums, reperfusion injury, keloid formation, scar tissue formation, pyrexia, periodontal disease, obesity, and radiation toxicity. In still another aspect, the IL-1 related disease is selected from the group consisting of Behcet's disease, ankylosing spondylitis, asthma, chronic obstructive pulmonary disorder (COPD), idiopathic pulmonary fibrosis (IPF), restenosis, diabetes, anemia, pain, a Crohn's disease-related disorder, juvenile rheumatoid arthritis (JRA), a hepatitis C virus infection, psoriatic arthritis, and chronic plaque psoriasis.

In the preferred embodiment, the IL-1 related disease is rheumatoid arthritis.

The method of the invention is particularly useful to predict the response to a treatment by anakinra in a patient with rheumatoid arthritis that is moderate or active. The disease activity can be measured according to the standards recognized in the art. The "Disease Activity Score" (DAS) is a measure of the activity of rheumatoid arthritis. In Europe the DAS is the recognized standard in research and clinical practice. The following parameters are included in the calculation (Van Gestel AM, Prevoo MLL, van't Hof MA, et al. Development and validation of the European League Against Rheumatism response criteria for rheumatoid arthritis. Arthritis Rheum 1996; 39:34-40).:
- Number of joints tender to the touch (TEN)
- Number of swollen joints (SW)
- Erythrocyte sedimentation rate (ESR)
- Patient assessment of disease activity (VAS; mm)

Patients with a disease activity score 28 (DAS28) ≥ 3.2 are a preferred group of patients.

Patients who are resistant to methotrexate (MTX), usually considered first-line therapy for the treatment of RA, are a further preferred group of patients for whom the method of the invention can be particularly useful.

More generally, patients who already receive a basic treatment for their IL-1 related disease, *e.g.* with MTX, azathioprine or leflunomide, are particularly good candidates for the test method of the invention.

After being tested for responsiveness to a treatment with anakinra, the patients may be prescribed with anakinra with or without the same basic treatment. In particular the combination anakinra/MTX can be particularly effective in patients with RA and other IL-1 related disease.

The term "biological sample" means any biological sample derived from a patient, preferably a sample which contains nucleic acids. Examples of such samples include fluids, tissues, cell samples, organs, biopsies, etc. Most preferred samples are blood, plasma, saliva, urine, seminal fluid, etc. Peripheral blood is preferred, and mononuclear cells (PBMCs) are the preferred cells. Total RNAs can be easily extracted therefrom. The biological sample may be treated prior to its use, *e̅.̅g̅.̅* in order to render nucleic acids available. Techniques of cell or protein lysis, concentration or dilution of nucleic acids, are known by the skilled person.

A "responder" patient, or group of patients, refers to a patient, or group of patients, who shows or will show a clinically significant relief in the disease when treated with anakinra. When the disease is RA, a preferred responder group of patients that provides for the control values is a group that shows a decrease of DAS28 ≥1.2 after three months of treatment with anakinra .

The expression level of the genes is determined and compared between a responder group and a non-responder group of patients. Said expression level of genes" correspond to the combined expression profile of said genes in either group.

The comparison between groups can be performed by computer tools, such as the supervised learning classification tool Support Vector Machine (SVM). These tools take into account the differential expression of the gene clusters, *i.e.* of the combination of the genes between groups, and generate an algorithm. The latter next allows for a prediction of a non-responder an responder status of any further patient, provided the same transcript level have been determined in said patient.

### The sets of Predictive genes

All the genes identified are known per se, and listed in the below tables A and B. Table A presents the set of 7 genes whose combined expression profile has been shown to be informative with regard to responsiveness to a treatment with anakinra. These are GTF2F2, CCT3, CROT, HNRPA3, ARL15, TMED5, and NRG3 transcripts.

**TABLE A: subset of 7 genes (transcripts)**

| **Gene** | ***UniGene access number*** | **Name** |
|---|---|---|
| GTF2F2 | Hs.654582 | General transcription factor IIF, polypeptide 2, 30kDa |
| CCT3 | Hs.491494 | Chaperonin containing TCP1, subunit 3 (gamma) |
| CROT | Hs.125039 | Carnitine O-octanoyltransferase |
| HNRPA3 | Hs.516539 | Heterogeneous nuclear ribonucleoprotein A3 |
| ARL15 | Hs.659125 | ADP-ribosylation factor-like 15 |
| TMED5 | Hs.482873 | Transmembrane emp24 protein transport domain containing 4 |
| NRG3 | Hs.125119 | Neuregulin 3 |

In a particular embodiment, the method of the invention further comprises determining the expression level of the genes of Table B:

**TABLE B : Other genes of interest for the predictive method**

| **Gene** | ***UniGene access number*** | **Name** |
|---|---|---|
| SERPINE1 | Hs.414795 | Serpin peptidase inhibitor, clade E, member 1 |
| MRPL40 | Hs.431307 | Mitochondrial ribosomal protein L40 |
| EIF3S12 | Hs.314359 | EIF3S12: Eukaryotic translation initiation factor 3, subunit 12 |
| OVGP1 | Hs.1154 | Oviductal glycoprotein 1, 120kDa (mucin 9, oviductin) |
| ZDHHC20 | Hs.564611 | Zinc finger, DHHC-type containing 20 |
| BAZ2B | Hs.470369 | Bromodomain adjacent to zinc finger domain, 2B |
| F11R | Hs.709404 | F11 receptor |
| SLC11A2 | Hs.505545 | Solute carrier family 11, member 2 |
| ONECUT1 | Hs.658573 | One cut domain, family member 1 |
| MAP4 | Hs.517949 | Microtubule-associated protein 4 |
| SLC15A4 | Hs.507260 | Solute carrier family 15, member 4 |
| CLEC2D | Hs.268326 | C-type lectin domain family 2, member D |
| RBM35B | Hs.592053 | RNA binding motif protein 35B |

### Determination of expression level

Determination of the expression level of a gene can be performed by a variety of techniques. Generally, the expression level as determined is a relative expression level.

More preferably, the determination comprises contacting the sample with selective reagents such as probes, primers or ligands, and thereby detecting the presence, or measuring the amount, of polypeptide or nucleic acids of interest originally in the sample. Contacting may be performed in any suitable device, such as a plate, microtiter dish, test tube, well, glass, column, and so forth In specific embodiments, the contacting is performed on a substrate coated with the reagent, such as a nucleic acid array or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable complex, such as a nucleic acid hybrid or an antibody-antigen complex, to be formed between the reagent and the nucleic acids or polypeptides of the sample.

In a preferred embodiment, the expression level may be determined by determining the quantity of mRNA.

Methods for determining the quantity of mRNA are well known in the art. For example the nucleic acid contained in the samples (*e.g.*, cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (*e. g.,* Northern blot analysis) and/or amplification (*e.g.*, RT-PCR). Preferably quantitative or semi-quantitative RT-PCR is preferred. Real-time quantitative or semi-quantitative RT-PCR is particularly advantageous.

Other methods of Amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (*e. g.* avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In a preferred embodiment, the invention provides an *in vitro* method for predicting whether a patient would be responsive to a treatment with a anakinra which method comprises determining the expression level of 7 genes in a biological sample of said patient, wherein said genes are GTF2F2, CCT3, CROT, HNRPA3, ARL15, TMED5, and NRG3, which method comprises the steps of providing total RNAs extracted from PBMCs obtained from a blood sample of the patient, and subjecting the RNAs to amplification and hybridization to specific probes, more particularly by means of a quantitative or semi-quantitative RT-PCR.

In another preferred embodiment, the expression level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, *e.g.* by using radioactive or fluorescent labelling. Many variants of the microarray hybridization technology are available to the man skilled in the art (see e.g. the review by Hoheisel, Nature Reviews, Genetics, 2006, 7:200-210)

In this context, the disclosure further provides a DNA chip comprising a solid support which carries nucleic acids that are specific to GTF2F2, CCT3, CROT, HNRPA3, ARL15, TMED5, and NRG3 genes.

Another subject of the disclosure is such DNA chip, which further carries nucleic acids that are specific to any or all of the genes of Table B.

Other methods for determining the expression level of said genes include the determination of the quantity of proteins encoded by said genes.

Such methods comprise contacting a biological sample with a binding partner capable of selectively interacting with a marker protein present in the sample. The binding partner is generally an antibody, that may be polyclonal or monoclonal, preferably monoclonal.

The presence of the protein can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound protein in a liquid phase from a solid phase support to which antigen-antibody complexes are bound. Solid supports which can be used in the practice of the invention include substrates such as nitrocellulose (*e. g.*, in membrane or microtiter well form); polyvinylchloride (*e. g.,* sheets or microtiter wells); polystyrene latex (*e.g.*, beads or microtiter plates); polyvinylidine fluoride; diazotized paper; nylon membranes; activated beads, magnetically responsive beads, and the like.

More particularly, an ELISA method can be used, wherein the wells of a microtiter plate are coated with an antibody against the protein to be tested. A biological sample containing or suspected of containing the marker protein is then added to the coated wells. After a period of incubation sufficient to allow the formation of antibody-antigen complexes, the plate (s) can be washed to remove unbound moieties and a detectably labeled secondary binding molecule added. The secondary binding molecule is allowed to react with any captured sample marker protein, the plate washed and the presence of the secondary binding molecule detected using methods well known in the art.

### Therapeutic applications

A method for treating a IL-1 related disease is contemplated, which method comprises
a) a preliminary step of testing whether a patient with a IL-1 related disease would be responsive to a treatment with a anakinra, by determining the expression level of 7 genes in a biological sample of said patient, wherein said genes are GTF2F2, CCT3, CROT, HNRPA3, ARL15, TMED5, and NRG3; hereby classifying the patient as responder or non responder;
b) a step of administering anakinra to a patient classified as responder.

The classification of the patient - as responder or non-responder- is made according to the combined expression level of said 7 genes. It allows to define a subgroup of patients who will be responsive to a treatment with a anakinra.

A further subject of the invention is then the use of anakinra for the preparation of a medicament for treating a patient with a IL-1 related disease, such as rheumatoid arthritis, which patient being classified as responder to a treatment with a anakinra, by determining the expression level of said eight genes in a biological sample of said patient.

The invention will be further illustrated by the following figures and examples. However, these examples and figures should not be interpreted in any way as limiting the scope of the present invention.

### EXAMPLE:

### Methods:

***Patients.*** A total of 32 patients, fulfilling the American College of Rheumatology (ACR) criteria for RA and followed in Rouen University Hospital were included in this study (Arnett FC. et al. 1988). The criteria for patient eligibility were: methotrexate treatment; disease activity score 28 (DAS28) ≥ 3.2 (van Gestel AM. et al. 1996); resistance to at least one conventional disease-modifying anti-rheumatic drug (DMARD) including methotrexate. Exclusion criteria were: evolving infectious disease; age <18 years; no contraception; pregnancy; cancer less than 5 years old; anakinra allergy. This protocol (numbered 2003/012) was approved by the North-West 1 Ethics committee (Haute-Normandie, France) and all participants signed an informed consent at time of enrolment. The study was declared to Clinical Trials (NCT00213538). For one month or more before the start of this study every patient was given fixed amounts of DMARD and nonsteroidal anti-inflammatory drug (NSAID) and did not receive any intra-articular steroid injection. During this study, every patient was given the same doses of methotrexate and prednisone as used before, and was treated with anakinra (Kineret ®, Amgen, France) as recommended by the manufacturer and the French Drug Agency AFSSAPS (100 mg/day by subcutaneous injection). Before the first injection of anakinra, DAS28, plasma C reactive protein (CRP) level, patient's assessment of pain (0-100 mm visual analogue scale, VAS), duration of morning stiffness, and physical function scored with the French version of the Health Assessment Questionnaire (HAQ) for RA were recorded (Guillemin F. et al. 1991). At 3 months, the patients were categorized as responders whenever a change of DAS28 ≥1.2 was obtained. All others were categorized as non-responders.

***PBMCs isolation and mRNA extraction and labelling.*** The PBMCs were isolated from venous blood by Ficoll-Hypaque centrifugation and total RNAs were extracted by Trizol Reagent® (Fischer Bioblock) according to the manufacturer instructions and quality was controlled on an Agilent 2100 Bioanalyzer (Agilent Technologies, Palo Alto, USA) and frozen at -80°C until further used. An internal, arbitrary standard was made of a mixture of total RNAs from PBMCs taken from 3 healthy donors. The oligodT-primed poly(A) mRNAs were labelled with [α³³P]dCTP as previously described, and the resulting, labelled cDNAs were immediately used for hybridization (Lequerré T. et al. 2006; Coulouarn C. et al. 2004).

***Cell culture.*** After gradient centrifugation with Ficoll, viable PBMCs from 7 healthy donors counted by trypan blue dye exclusion (2.10⁷ cells/well) were cultured in RPMI 1640 (Fischer Biolock) supplemented with 2 mM glutamine (Invitrogen), 50 UI/ml penicillin G (Invitrogen), 50 µg/ml streptomycin (Invitrogen) and 1% heat inactivated fetal calf serum (Invitrogen). Cells were enriched at time 0 with 10 ng/ml IL-1 (Promokine) and/or 3 ng/ml IL-1Ra (Promokine), as recommended by the manufacturer. They are incubated at 37°C, 5% CO₂ during 30 min, 60 min, 90 min, 6 hours and over-night. A negative control well was cultured per individual in RPMI-1640 alone. Cells were resuspended in 1 ml Trizol Reagent® (Fischer Bioblock) and frozen at -20°C until extraction of mRNA.

***Transcriptome analysis and qRT-PCR.*** Our array covering 12,000 cDNA probes for 10,000 non-redundant genes and various negative controls as well as nylon arraying of PCR-amplified probes and hybridization of [α³³P]dCTP-labelled mRNAs have all been extensively described and validated in a previous report (Coulouarn C. et al. 2004). Briefly, cDNA probes selected on the basis of a tissue-preferred expression in liver corresponded to genes with a liver-restricted expression (10% of the probes) as well as genes with a hepatic expression along with a broad expression in some (50%) or many non-hepatic tissues (40%) (Coulouarn C. et al. 2004). All arrays were made from a single batch of cDNA probes. Every RNA sample was hybridized at least twice on separate arrays. Whenever necessary, the sequence of cDNA probes was controlled with an ABI3100 capillary sequencer (Applied Biosystems, Foster City, USA). Real-time, quantitative reverse transcription PCR (qRT-PCR) of mRNAs were performed with a Light Cycler (Roche Diagnostics, Manheim, Germany) and normalization with the 18S RNA amount were done in duplicate as described and the primers designed with the Primer3 software (http://frodo.wi.mit.edu) are listed in Table 1 (Coulouarn C. et al. 2004).

**Table 1.Primers used for qualitative RT-PCR**

| **Gene** | **Forward** | **Reverse** |
|---|---|---|
| NRG3 | 5'-CGCGGGGCTTAATACATAGG-3' (SEQ ID NO:1) | 5'-GTACCTGCTCGGCATGGAGT-3' (SEQ ID NO:2) |
| MRPL40 | 5'-GGCAGACGCAGCTTAGAGAG-3' (SEQ ID NO:3) | 5'-CCGCTCTCTTGCTTTATCCA-3' (SEQ ID NO: 4) |
| ONECUT1 | 5'-CATGGTGACTGGAAACATGC-3' (SEQ ID NO: 5) | 5'-GATCTGCACTTGGACAGCAA-3' (SEQ ID NO:6) |
| OVGP1 | 5'-CCCCTCTTCTCTCTGCCTGA-3 (SEQ ID NO: 7) | 5'-GGCCTGCAACCCATTCTTAG-3' (SEQ ID NO: 8) |
| ZDHHC20 | 5'-CGGATTGTTGTGATGACTGC-3' (SEQ ID NO:9) | 5'-TGTCATTTCCTTCTGCCTGA-3' (SEQ ID NO:10) |
| GTF2F2 | 5'-CCTGGCCCTCAGAGATTTTG-3' (SEQ ID NO: 11) | 5'-AAACGGCAATCAGGTCGAAT-3' (SEQ ID NO: 12) |
| SERPINE 1 | 5'ACAGGAGGAGAAACCCAGCA--3' (SEQ ID NO: 13) | 5'-GAAGAAGTGGGGCATGAAGC-3' (SEQ ID NO:14) |
| MAP4 | 5'-CCACCTCCGATCATCAGTCA-3' (SEQ ID NO:15) | 5'-GGTGAGCAGCAGGTGAACAG-3' (SEQ ID NO:16) |
| CROT | 5'-ACCTGATGGACCTGGGATTG-3' (SEQ ID NO:17) | 5'-TTTGTCACCCCAGCGTACTG-3' (SEQ ID NO:18) |
| SLC11A2 | 5'-TGCGGAGCTGGTAAGAATCA-3' (SEQ ID NO:19) | 5'-CCCAGGGGACTGTGAAAGAG-3' (SEQ ID NO:20) |
| EIF3S12 | 5'-TTTTGTACCTCGGGGACCTG-3' ((SEQ ID NO:21) | 5'-GCCGTATTTGCTCATCCACA-3' (SEQ ID NO:22) |
| BAZ2B | 5'-AACAGGCTCGGGTTGCTAAA-3' (SEQ ID NO:23) | 5'-CATTTGCCGCTTCTTCCTTC-3' (SEQ ID NO:24) |
| ARL15 | 5'-TAAAGCAGGAGCCGGTCTGT-3' (SEQ ID NO:25) | 5'-GGGGTCTTGTCTGCCTTCAC-3' (SEQ ID NO:26) |
| SLC15A4 | 5'GCTCATCCTCCTGCTCATCC--3' (SEQ ID NO:27) | 5'-CGTTGCCGATGGTCTGATTA-3' (SEQ ID NO:28) |
| F11R | 5'-GCGCAAGTCGAGAGGAAACT-3' (SEQ ID NO:29) | 5'-ACTCCACACGGGGAGAAGAA-3' (SEQ ID NO:30) |
| CCT3 | 5'-GCGTGAATCCGGAAGAAAAG-3'(SEQ ID NO:31) | 5'-CTGTTGTGGTCCCATCTCCA-3' (SEQ ID NO:32) |
| TIMED5 | 5'-TGGGAGAACAGGCACAAGAA-3' (SEQ ID NO:33) | 5'-GACACCACCACCATGACCAC-3' (SEQ ID NO:34) |
| HNRPA3 | 5'-AATCCCAGCACTTTCGGAGA-3' (SEQ ID NO:35) | 5'-AAGCGATTCTCCTGCCTCAG-3' (SEQ ID NO:36) |
| CLEC2D | 5'-ATGCCTCAGCCTCCCAAATA-3' (SEQ ID NO:37) | 5'-AGGCCCCAGGATATGCTTTT-3' (SEQ ID NO:38) |
| RBM35B | 5'-CTGCTCAGCGTTGCCATAAG-3' (SEQ ID NO:39) | 5'-GGCTTGGAAGGTGGTGTAGG-3' (SEQ ID NO:40) |
| 18S | 5'-GTGGAGCGATTTGTCTGGTT-3' (SEQ ID NO:41) | 5'-CGCTGAGCCAGTCAGTGTAG-3' (SEQ ID NO:42) |

***Image analysis and data mining.*** Image analysis with the XDotsReader software (version 1.8; COSE, Le Bourget, France), subtractions of noise and spot background, and image normalization with the median absolute deviation of all signals per image were performed exactly as previously detailed (Fundel K. et al. 2008). A transcript was considered to be expressed if at least two hybridizations provided a positive signal. Statistical analyses of clinical data were done with the GraphPad Instat software, version 3 (http://www.graphpad.com). The TIGR Multiexperiment viewer (Tmev version 2.2; http://www.tm4.org) was used for i) unsupervised hierarchical clustering using the Manhattan distance and complete linkage options, ii) leave-one-out cross-validation with the supervised learning classification tool Support Vector Machine (SVM) and iii) the supervised statistical one-class t-test with the adjusted Bonferroni's correction. Protein networks were identified with Bibliosphere (www.geomatix.de) with different levels of stringency (B1: two genes are co-cited in a same sentence; B2: level B1 restricted to sentences with a function word; B3: level B2 restricted to sentences with order *"*gene ..*function word*....gene"). Information about our clinical and experimental data complies with the recommendations for a Minimum Information About Microarray Experiments (MIAME) and the raw data have been deposited (accession number GSE3592) in the GEO repository (http://www.ncbi.nlm.nih.gov/projects/geo).

### Results:

***RA patients and response to anakinra.*** Two sets of responders (R1 to 15) or non-responders (NR1 to 17) to an anakinra/methotrexate combination were so categorized at 3 months with the European League Against Rheumatism (EULAR) criteria, as recommended (van Gestel AM. et al. 1996). Tables 2 and 3 provide demographic and clinical information for these 32 patients, at entry and at 3 months. The average disease duration was 11.5 ± 9 (mean ± SD) years and the DAS28 score indicated that all these patients had a high level of RA activity (5.4 ± 1), which fits with their resistance to one or more DMARDs. More accurately, mean DAS28 was 5.5 ± 0.9 and 5.2 ± 1.3 in responders and non responders respectively. Before treatment, all variables (but pain on VAS), including DAS28, were similar in responders *versus* non-responders and in subset 1 *versus* 2. Following treatment, the DAS28 score significantly improved at 3 and/or 6 months in responders (average decrease DAS28: 2.3 at 3 months and 2.7 at 6 months) whereas it remained high in non-responders (average decrease DAS28: 0.4 at 3 months and 0.3 at 6 months). Within each set of responders or non-responders, the patients were randomly separated as a training subset (subset 1) for transcriptome analysis and a validation subset (subset 2) for qRT-PCR. At this stage, we paid attention to retaining a relatively large number of patients in subset 2. As noted in Tables 2 and 3, most features did not significantly differ between paired subsets 1 and 2. Among responders, 6 patients are still treated with anakinra (after 43 months in average), 3 patients stopped anakinra because of pregnancy for 1 patient and side effects for 2 patients (allergy and bronchitis) and 6 patients escaped to anakinra after 27 months in average of treatment.

***Gene profiling in pre-treatment PBMCs correlates with treatment responsiveness.*** Gene profiling in PBMCs was studied in the two training subsets 1 from responders and non-responders (total, 14 patients). On average, 5967 ± 1418 transcripts were detected in PBMCs, with 85% overlap in transcript identities between responders and non-responders. To precisely identify transcripts that were differentially regulated in responders *versus* non-responders, we selected every transcripts whose variation between responders and non-responders was statistically significant by t test adjusted with Bonferroni's correction. This resulted in 51 different transcripts (*p* < 1.10⁻⁴) which the most of them are up regulated in non-responders and down regulated in responders. These transcripts are listed and detailed in Table 4 which included 52 transcripts because *STIP1* is redundant. The identity of the corresponding microarray cDNA probes was verified by sequencing. Finally, we performed an unsupervised hierarchical clustering of the 14 patients above (subsets 1). This was based on the levels of the 51 transcripts indicated above, which resulted in a perfect separation of the responders and non-responders into two major clusters. With the PBMCs from others patients (n = 18), we wished to confirm that a combination of the above transcript levels could be used as a predictor of responsiveness. For this purpose, we aimed at measuring the levels of the above 51 transcripts by qRT-PCR and comparing them between our two validation subsets 2 (responders or non-responders, total 18 patients). However, among these 51 transcripts, 13 putative transcripts were merely identified by one IMAGE clone without knowledge of the intron/exon structure and hence they were not retained in our approach. Moreover, among the 38 remaining transcripts, 6 of them failed to provide reliable data by qRT-PCR, despite repeated attempts with various primers. Eventually, 32 out of our 51 transcripts could be reliably quantified by qRT-PCR. Among these 32 transcripts, only the 20 transcripts whose *p* value was < 1.10⁻⁴ and with the higher divergence of gene expression level between responders and non responders were selected: GTF2F2, CCT3, CROT, HNRPA3, ARL15, TMED5, NRG3, SERPINE1, MRPL40, EIF3S12, OVGP1, ZDHHC20, BAZ2B, F11R, SLC11A2, ONECUT1, MAP4, SLC15A4, CLEC2D, and RBM35B. An unsupervised hierarchical clustering of the 18 patients in subsets 2, as based upon these 20 transcript levels, resulted in two major clusters of responders *versus* non-responders, with 3 (17%) misclassified patients (R10, NR9, NR11). Despite being informative, such a hierarchical clustering lacks statistical power, and hence the efficiency of the above set of 20 transcripts for patient classification was thus further evaluated by leave-one-out cross-validation. The resulting data indicate that this set of transcripts provides 80% sensitivity and 87.5% specificity for identification of responders and non-responders (Table 5). To determine the minimal number of transcripts that should be measured for an acceptable prediction of responsiveness, we tested in the 18 patients from the subsets 2 above a series of combinations of transcripts, and we varied the number and identity of the transcripts actually used. With a given set of only 7 transcripts (GTF2F2, CCT3, CROT, HNRPA3, ARL15, TMED5, and NRG3), 15 out of 18 patients could be correctly classified as responders or non-responders by hierarchical clustering. Finally, leave-one-out cross-validation (Table 5) indicated that a given set of 7 transcripts as a predictor of responsiveness was at least as good as the set of 20 transcripts above.

***The combination of transcripts suits an IL-1β signature.*** We investigated the function of each gene belonging to the combination able to predict anakinra/methotrexate responsiveness. First, Bibliosphere allows analyzing gene/gene, and gene/transcription factor relations from their co-citation in PubMed abstracts with different threshold of stringency. Among the 43/51 transcripts with a gene symbol (for 8 genes, any information about intron/exon sequence was available) which were download in BiblioSphere software, 34/43 genes passed the software filter and were input for further analysis. So, inspection of the literature associated with these 34 input genes selected by BiblioSphere and 2 genes co-cited (IL-1β, IL-1Ra) revealed a strong relation between *IL*-1β and 76% among them (*i.e.* 26/34 genes) with a B1 level of stringency, 64 % of them (i.e. 22/34) with a B2 level of stringency and 56 % among them (*i.e.* 19/34) with a B3 level of stringency. The gene network was built around IL-1β with either direct connection between genes, either indirect links through a transcription factor. This suggested a regulation of these genes by one (or more) IL-1β dependant pathway. Later on, the high percentage of genes regulated directly or indirectly by IL-1β and belonging to the combination was in favour of an IL-1β signature. However, if we considered the smallest combination described above, only *GTF2F2* was linked to IL-1β by BiblioSphere. As expecting, any data was found between IL-1β and the 6 others genes (*CCT3*, *CROT, HNRPA3, ARL15, TMED5, NRG3)* in Pubmed database. We aimed to prove a modulation of these genes by IL-1β or IL-1Ra. From *in vitro* healthy PBMCs cultures, we measured at different time the IL-1 and IL-1Ra effects on gene expression for the 7 genes (minimal combination) able to discriminate the responders and non-responders. As positive control, we used *COX2* and *EP4* genes and checked that IL-1 increased the *COX2* expression and decreases the *EP4* expression. We cultured fresh healthy PBMCs with and without IL-1β (or IL-1Ra) and measured the gene expression level of these 7 genes by qRT-PCR. The gene expression level decreased significantly for 3/7 (*ARL15*, *CROT*, *CCT3*) at 1 h30 and/or 6 hours in presence of IL-1β. IL-1Ra alone and both IL-1/IL-1Ra had no significant effect on the gene expression level. These results suggested also that these genes were modulated by IL-1. Taken together, the gene regulation by IL-1β was demonstrated for 4/7 genes (*CCT3*, *CROT*, *GTF2F2* and *ARL15*) of the minimal combination able to predict the anakinra responsiveness. This argues for an IL-1 signature able to predict the anakinra response.

### Discussion:

Despite of increasing therapeutic options, a great number of RA patients will achieve a good clinical response, the prescription of these drugs is still empiric for a given patient. Moreover, considering the potential serious side effects of these drugs, their high cost and their failure in 30 to 40% patients according the clinical studies, the identifying predictors of therapeutic response would be of helpful value in the RA therapeutic strategies. That's why we have developed a program to identify true predictive biomarkers for the responsiveness to biotherapy in RA. A small set of biological markers usually used for RA diagnosis or prognosis is unable to predict individual responsiveness to TNFα blocking agents (Lequerré T. et al. 2007). In addition, the few studies that used these markers have relied on the differences in protein markers measured at baseline *versus* some weeks after treatment onset. These studies required exposure of every patient to treatment and were irrelevant with a truly predictive approach which consists to identify biomarkers without *a priori* before the treatment was prescribed to a given patient. Therefore, to enable such a prediction, global approaches based on proteomics or transcriptomics have been recently considered (Drynda S. et al. 2004). However, except our previous established combination of transcripts able to predict the infliximab/methotrexate responsiveness in the context of RA, a very few informative transcripts or proteins have been identified (Lequerré T. et al. 2006). So, we aimed to identify a list of transcripts could be related to anakinra/methotrexate responsiveness without any exposure treatment, which enables to be restricted to responders.

Three months of treatment was chosen as the endpoint of our study, as recommended by international experts because the objective of RA treatment is a rapid response. If this early evaluation at three months discloses a moderate or absent response, this procedure allows another treatment to be used as early as possible. Also, the level of the response was the improvement of the DAS28 > 1.2 as recommended by the international expert when the study was designed. Nevertheless, the DAS28 improved in responders from 5.5 to 3.2, *i.e.* the up-threshold of the minimal disease activity according the DAS28 score (van Gestel AM. et al. 1996). However, the improvement in responders group is in agreement with the new objectives of RA treatment, *i.e.* at least the minimal disease activity. Moreover, among the 15 responders, the responsiveness for six patients still treated by anakinra did not vary over the 43 months of following. Six patients escaped to anakinra but after in average 27 months. The list of transcripts identified above is efficient to predict the responsiveness or the primary failure to anakinra but not the secondary failure. Indeed, to predict this later, *i.e.* the graduated escape in the duration, is another question for which the study was not designed. Gene expression was measured in PBMCs because this is an acknowledged, non-invasive procedure for diagnosis or prognosis of auto-immune disease (Olsen NJ. et al. 2004). Specifically, in a RA context, PBMCs as a surrogate tissue are advantageous as they allow for a screening in any subject, whereas synovium is amenable to analysis in few patients. Also, we have analyzed the PBMC transcriptome with an arbitrary collection of approximately 10,000 cDNA probes (Coulouarn C; et al. 2004). Since this restrictive procedure cannot measure every transcript expressed in the PBMCs, it does not intend to provide a genome-wide view of the RA-associated gene dysregulations in this tissue. Yet, this approach is quite acceptable when inferring prognosis from gene profiling is the major task. Overall, the present study was not primarily designed to increase our understanding of RA physiopathology but it was mostly suited to a predictive usage of some combined transcript levels. Eventually, our data illustrate that a non-invasive transcriptome analysis done in PBMCs with an array of probes devoid of a specific selection towards the disease under study enables an efficient prediction of treatment responsiveness. For a second time, we demonstrated that our tool and our approach, applied at two different drugs, are helpful and powerful to identify and validate a combination of transcripts able to predict the response to a drug.

By very stringent *t* test (adjusted Bonferroni's correction and *p* < 1 10⁻⁴), we have identified a short list of 51 transcripts whose combined expression levels in PBMCs are an efficient discriminator of responders *versus* non-responders to anakinra/methotrexate. Indeed, Bonferroni's correction has been recognized as a drastic one when used in this context (Allison DB. et al. 2006). Measuring by qRT-PCR the 20 transcript levels among these 51 indicated that their performance as a predictor of responsiveness was equal to that obtained with 51 transcripts. Ultimately, a given combination of 7 selected transcripts as a predictor of responsiveness was as powerful as any higher number of transcripts. This observation that a given combination of very few transcripts can equal or even outperform the predictive strength of a higher number of transcripts has also been reported in another context, namely the response to hepatitis C treatment (Chen L. et al. 2005). This small size for an informative gene set is most encouraging when the need comes for the development of a reliable, fast and cheap assay for measures of informative transcript levels in a clinical setting.

Among the 51 transcripts, and more exactly the 34 genes passing the filter of BiblioSphere, 56% of them are related with IL-1β either through a transcription factor either directly. Although these relations are not sole with IL-1β, the high proportion of gene linked to IL-1β in the combination lead us to consider this combination as an IL-1β signature. Some genes are regulated by transcription factors as NFκB1 (*SLC11A2*, *MCM3AP*), STAT3 (*LEPR*), CEBPβ (*RUNXIT1*, *ELF2*), JUN (*BST2*), MYC (*GTF2F2*). Once IL-1 links on its receptor, the IL-1 signalling pathway activates these transcription factors such as NFκB or CEBPβ which regulate in their turn the genes under their control. IL-1β controls also the B cells and T cells functioning through the activation of genes such as *ICOSL* involved in the co-stimulation of T cells or *BST2* that facilitated the pre-B-cell growth. *CLEC2D* or *TTR* are involved in the bone and cartilage metabolism since *CLEC2D,* also known osteoclast inhibitory lectin (OCIL), limits the osteoclast formation and inhibits bone resorption while TTR is produced by human chondrocytes after stimulation by IL-1β and oncostatin M. Moreover, *GTF2F2* involved in both specific initiation and elongation of RNA synthesis by RNA polymerase and *STIP1*, an extracellularly co-chaperone adaptor protein for Hsp70/Hsp90 leading to the activation of several signal transduction pathways, some of which modulate cell survival, are found as autoantigens in RA. Indeed, *GTF2F2* is a direct functional target of Fos and Jun to initiate the transcription through IL-1β signalling pathway. STIP-1 was also identified as an auto-antigen by Western blot approach in the serum of 111 early untreated RA patients. Leptin receptor belongs to the class I cytokine receptor superfamily (including receptors for IL-6, leukaemia inhibitory factor, oncostatin M and gp130) and activates the JAK-STAT pathway. Leptin receptor is expressed by CD4+ and CD8 +T cells. Leptin, the ligand's leptin receptor, is produced by adipocyte and involved in CD4⁺ T lymphocyte proliferation, cytokine secretion, angiogenesis. Moreover, B cell express leptin receptor mRNA, indicating that in addition to its effect on T lymphocyte responses, leptin may exert a direct effect on B cells and contributes to the mechanisms of joint inflammation in Ag-induced arthritis by regulating both humoral and cell-mediated immune responses. Circulating leptin is also increased under inflammatory conditions, both in acute and in chronic inflammation diseases such RA. In our study, the level of leptin receptor gene expression is less high in responders and non responders that is compatible with the murin model leptin-deficient mice which developed less severe arthritis compared with control mice. *ABCC5,* also known, multidrug resistance proteins, is a member of the ATP-binding cassette superfamily of membrane transporters that mediate the ATP-dependent transport of various substrates across biological membranes. *ABCC5* is especially a transporter of (anti)folates and contributes to resistance against antifolate drugs. This gene was down-regulated in responders in comparison with non-responders indicating that this combination of gene predicts probably the response to the association methotrexate/anakinra and not only to anakinra. Any other link between these transcripts and IL-1β or RA was found in the literature.

If we consider the smallest combination of genes, only *GTF2F2* was associated with IL-1β by Bibliosphere investigation. As indicated above, it has been identified as an auto-antigen in RA. So, we investigated *in vitro* the 6 others genes of the small set of transcripts to known if they are regulated or not by IL-1β or IL-1Ra. From healthy PBMCs cultured *in vitro* with only IL-1β or IL-1Ra or both, we demonstrated for the first time that *CROT*, *CCT3* and *ARL15* expression was down-regulated by IL-1β. *CCT3*, also called chaperonin containing TCP-1 or TCP-1 Ring complex, is an ATP-dependent chaperone playing an important role in the folding of cytoskeletal components. Elevated CCT3 expression possibly impairs correct folding and assembly of complex proteins. Regarding *ARL15,* this is an ADP-ribosylation protein involved in nuclear factor kappa B-dependent gene expression induced by lipopolysaccharide in murine macrophages. *ARL15* participates to activate NF-κB and could be regulated by. *CROT* is involved in fatty acid metabolism. *HNRPA3* is an abundant nuclear factor that binds Pol II transcripts and regulates RNA splicing or transcription. *TMED5* belongs is a type I transmembrane protein involved in secretory protein transport from the endoplasmic reticulum to the Golgi complex. *NRG3* is a neuregulin protein contributing to the cell-cell signaling. It is a ligand for receptor tyrosine kinases of the ErbB family. *NRG3* is at least expressed in mammary gland, including breast cancer and is involved in oligodendrocyte survival. All these genes are down-regulated in responders in comparison with non-responders to anakinra in RA pateints. To prove a regulation of these genes by IL-1β, *in vitro* experiments with healthy PBMCs were performed for 7 genes. We demonstrated for the first time an IL-1β regulation for *CCT3*, *CROT* and *ARL15* since IL-1β regulates negatively these genes. However, the gene expression level was studied with healthy PBMCs that was not reproduced exactly the RA environment.

The combined levels of a small set of discriminative transcripts, as an IL-1β signature, have provided for the first time a tool for prediction of anakinra/methotrexate efficacy in patients with long standing and very active disease. It remains to be seen whether our predictors can prove useful in patients with recent disease. Nevertheless, this work validated a second time, after the infliximab study, our approach to identify biomarkers able to predict the drug responsiveness (Lequerré T. et al. 2006). This approach, extending to others molecules, should help soon the rheumatologist to optimize the prescription of these drugs for a given patient. Ultimately, we anticipate that a small series of parallel tests for such drug specific combinations of transcripts, as quantified on a specifically designed DNA chip, should routinely allow one to select the most appropriate treatment for every RA patient, with the resulting and beneficial eradication of the non-responder or moderate responder phenotype.

**Table 2. Demographic and clinical data of RA patients at entry of study.**

| | **Responders**^{A} | | **Non-responders** | |
|---|---|---|---|---|
| **Parameter** | **subset 1**^{B} (n = 7) | **subset 2** (n = 8) | **subset 1** (n = 7) | **subset 2** (n = 10) |
| **age** (years) | 49.6 ± 19.4^{C} | 53.9 ± 10.9 | 56.7 ± 11.8 | 55.7 ± 9.5 |
| **sex** (men/women) | 0/7 | 2/6 | 1/6 | 1/9 |
| **RA duration** (years) | 8 ± 5.7 | 12.5 ± 8.9 | 17.9 ± 12.7 | 8.7 ± 6.6 |
| **methotrexate** (mg/week)^{D} | 13.2 ± 5.7 | 12.8 ± 6.2 | 12.5 ± 5 | 14.5 ± 4.5 |
| **prednisone** (mg/day) | 4.4 ± 4.3 | 2.7 ± 3.5 | 5.7 ± 8.4 | 6.5 ± 6 |
| **patients with NSAIDs**^{E} | 4 | 3 | 4 | 5 |
| **patients with rheumatoid factor** | 4 | 8 | 4 | 7 |
| **patients with anti-CCP abs**^{F} | 4 | 7 | 5 | 8 |

| | | | | |
|---|---|---|---|---|
| ^{A}categorized as indicated in Patients and Methods. ^{B}transcript levels were measured by microarray in subsets 1 or qRT-PCR in subsets 2. ^{C}mean ± SD. ^{D}maximally tolerated dose in a given patient. ^{E}non-steroidal anti-inflammatory drugs. ^{F}anti-cyclic citrullinated peptide antibodies. In this table, all comparisons were non significant (Mann and Whitney's non parametric test). | | | | |

**Table 3. Clinical data at baseline and at 3 months**

| **Responder^{A}** | | | | | | | |
|---|---|---|---|---|---|---|---|
| **Non-responders** | | | | | | | |
| | | | **subset 1** | | **subset 2** | | **subset 1** |
| | | | | **subset 2** | | | |
| | | | **baseline** | **3 months^{a}** | **baseline** | **3 months** | |
| | | | **baseline** | **3 months** | **baseline** | **3 months** | |
| **Morning stiffness (min)** | | | 59 ± 63^{B} | 28 ± 43 | 153 ± 119 | 53 ± 86* | 54 |
| ± 47 | 28 ± 27 | 92 ± 118 | 43 ± 55 | | | | |
| **DAS28^{C}** | | | 5.5 ± 0.3 | 3.3 ± 0.8* | 5.6 ± 1.1 | 3.2 ± 1.2* | 4.9 ± |
| 1.0 | 4.4 ± 1.7 | 5.4 ± 1.4 | 5.0 ± 1.2 | | | | |
| **Pain** (0-100 mm VAS)^{D} | | | 77.1 ± 18.9 | 41.4 ± 15.7* | 63.1.±22.2 | 37.7 ± 23.0 | 41.3 ± |
| 17.7** | 40.1 ± 31.7 | 65.0 ± 21.9 | 56.3 ± 22.1 | | | | |
| **ESR** (mm/hour)^{E} | | | 34.6 ± 8.4 | 14.7 ± 9.9* | 38 ± 29.1 | 14.2 ± 13.4* | 25.6 ± |
| 22.8 | 19.1 ± 18.2 | 41 ± 28.7 | 29.2 ± 24.7* | | | | |
| **CRP** (mg/L)^{F} | | | 21.3 ± 11.1 | 6.1 ± 2.7* | 26.5 ± 30.6 | 7.6 ± 4* | 22.4 ± |
| 29.6 | 17.4 ± 25.2 | 28.8 ± 25.9 | 14.8 ± 18.3* | | | | |
| **HAQ score** (0-3 scale)^{G} | | | 1.8 ± 0.5 | 1.2 ± 0.6 | 1.4 ± 0.7 | 0.8 ± 0.6 | 1.8 ± |
| 0.6 | 1.7 ± 0.7 | 1.5 ± 0.5 | 1.5 ± 0.4 | | | | |

| | | | | | | | |
|---|---|---|---|---|---|---|---|
| ^{A}*i.e*.Respo nse assessed after 3 months under anakinra/methotrexate. ^{B}mean ± SD. Significant differences between groups are noted as follows: * difference at baseline *versus*3 months in this subset (p<0.05, paired Wilcoxon's test); ** difference between responders *versus*non - responders at baseline (p<0.05. Mann and Whitney's test). All other comparisons were non-significant. ^{C}disease activity score. ^{D}patient's assessment of pain. ^{E}ESR : erythrocyte sedimentation rate; CRP : C-reactive protein; ^{G}HAQ : health assessment questionnaire. | | | | | | | |

**Table 4: Transcripts as predictors of anakinra responsiveness**

| ID clone^{A} | Encoded protein | Symbol^{B} | Gene localisation | *p*value ^{C} |
|---|---|---|---|---|
| Hs.368431 | Runt-related transcription factor 1; translocated to, 1 (cyclin D-related) | RUNX1T1 | 8q22 | < 1.10⁻⁴ |
| Hs.531561 | Epithelial membrane protein 2 | EMP2 | 16p13.2 | < 1.10⁻⁴ |
| Hs.125039 | Carnitine O-octanoyltransferase | **CROT** | 7q21.1 | < 1.10⁻⁴ |
| Hs.337295 | Stress-induced-phosphoprotein 1 (Hsp70/Hsp90-organizing protein) | STIP1 | 11q13 | < 1.10⁻⁴ |
| Hs.405880 | Mitochondrial ribosomal protein S21 | MRPS21 | 1q21 | < 1.10⁻⁴ |
| Hs.507260 | Solute carrier family 15, member 4 | **SLC15A4** | 12q24.32 | < 1.10⁻⁴ |
| Hs.491494 | Chaperonin containing TCP1, subunit 3 (gamma) | CCT3 | 1q23 | < 1.10⁻⁴ |
| Hs.23581 | Leptin receptor | LEPR | 1p31 | < 1.10⁻⁴ |
| Hs.431307 | Mitochondrial ribosomal protein L40 | **MRPL40** | 22q11.21 | < 1.10⁻⁴ |
| Hs.482873 | Transmembrane emp24 protein transport domain containing 4 | **TMED5** | 1 pter-q31.3 | < 1.10⁻⁴ |
| Hs.654582 | General transcription factor IIF, polypeptide 2, 30kDa | **GTF2F2** | 13q14 | < 1.10⁻⁴ |
| Hs.517949 | Microtubule-associated protein 4 | **MAP4** | 3p21 | < 1.10⁻⁴ |
| Hs.382306 | CDK8 gene for cyclin-dependent kinase | CDK8 | 13q12 | < 1.10⁻⁴ |
| Hs.153026 | switch-associated protein 70 | SWAP70 | 11p15 | < 1.10⁻⁴ |
| Hs.132526 | IMAGE3625232 | n.a. | n.a. | < 1.10⁻⁴ |
| Hs.389037 | Minichromosome maintenance complex component 3 associated protein | MCM3AP | 21q22.3 | < 1.10⁻⁴ |
| Hs.517517 | E1A binding protein p300 | EP300 | 22q13.2 | < 1.10⁻⁴ |
| Hs.482730 | EGF-like repeats and discoidin I-like domains 3 | EDIL3 | 5q14 | < 1.10⁻⁴ |
| Hs.659125 | ADP-ribosylation factor-like 15 | **ARL15** | 5p15.2 | < 1.10⁻⁴ |
| Hs.659718 | E74-like factor 2 (ets domain transcription factor) | ELF2 | 4q28 | < 1.10⁻⁴ |
| Hs.14155 | Homo sapiens inducible T-cell co-stimulator ligand | ICOSLG | 21 q22.3 | < 1.10⁻⁴ |
| Hs.314359 | EIF3S12: Eukaryotic translation initiation factor 3, subunit 12- | **EIF3S12** | 19q13.2 | < 1.10⁻⁴ |
| Hs.592243 | THO complex 2 | THOC2 | Xq25-q26.3 | < 1.10⁻⁴ |
| Hs.109929 | GRIP1 associated protein 1 | GRIPAP1 | Xp11 | < 1.10⁻⁴ |
| Hs.414795 | Serpin peptidase inhibitor, clade E, member 1 | **SERPINE1** | 7q21.3-q22 | < 1.10⁻⁴ |
| Hs.513071 | Mesoderm development candidate 1 | MESDC1 | 15q13 | < 1.10⁻⁴ |
| Hs.270845 | Kinesin family member 22 | KIF23 | 151q23 | < 1.10⁻⁴ |
| n.a. | RP11-430H15 | n.a. | n.a. | < 1.10⁻⁴ |
| n.a. | ATP-binding cassette, sub-family C (CFTR/MRP), member 5 | ABCC5 | 3q27 | < 1.10⁻⁴ |
| Hs.300774 | fibrinogen, B beta polypeptide (FGB) | FGB | 4q28 | < 1.10⁻⁴ |
| n.a. | RP11-372K14 | n.a. | n.a. | < 1.10⁻⁴ |
| Hs.470369 | Bromodomain adjacent to zinc finger domain, 2B | **BAZ2B** | 2q23-q24 | < 1.10⁻⁴ |
| Hs.125119 | Neuregulin 3 | **NRG3** | 10q22-q23 | < 1.10⁻⁴ |
| Hs.505545 | Solute carrier family 11, member 2 | **SLC11A2** | 12q13 | < 1.10⁻⁴ |
| Hs.118110 | Bone marrow stromal cell antigen 2 | BST2 | 19p13.2 | < 1.10⁻⁴ |
| Hs.356626 | Hypothetical protein P117 | n.a. | 19p13.3 | < 1.10⁻⁴ |
| Hs.508266 | COMM domain containing 6 | COMMD6 | 13 | < 1.10⁻⁴ |
| Hs.427202 | Transthyretin (prealbumin, amyloidosis type I) | TTR | 18q12.1 | < 1.10⁻⁴ |
| Hs.592053 | RNA binding motif protein 35B | **RBM35B** | 16q22.1 | < 1.10⁻⁴ |
| Hs.709404 | F11 receptor | **F11R** | 1q21.2-q21.3 | < 1.10⁻⁴ |
| Hs.1154 | Oviductal glycoprotein 1,120kDa (mucin 9, oviductin) | **OVGP1** | 1p13 | < 1.10⁻⁴ |
| Hs.647584 | Hypothetical protein LOC196913 | n.a. | 14q22.1 | < 1.10⁻⁴ |
| Hs.564611 | Zinc finger, DHHC-type containing 20 | **ZDHHC20** | 13q12.11 | < 1.10⁻⁴ |
| Hs.268326 | C-type lectindomain family 2, member D | **CLEC2D** | 12p13 | < 1.10⁻⁴ |
| Hs.658573 | One cut domain, family member 1 | **ONECUT1** | 15q21.1-q21.2 | < 1.10⁻⁴ |
| Hs.516539 | Heterogeneous nuclear ribonucleoprotein A3 | **HNRPA3** | 2q31.2 | < 1.10⁻⁴ |
| Hs.76206 | Cadherin 5, type 2, VE-cadherin (vascular epithelium) | CDH5 | 16q22.1 | < 1.10⁻⁴ |
| n.a. | RP5-1007H16 | n.a. | n.a. | < 1.10⁻⁴ |
| n.a. | IMAGE205927 | n.a. | n.a. | < 1.10⁻⁴ |
| Hs.188757 | Potassium channel tetramerisation domain containing 20 | KCTD20 | 6p21.31 | < 1.10⁻⁴ |
| n.a. | IMAGE428468 | n.a. | n.a. | < 1.10⁻⁴ |

| | | | | |
|---|---|---|---|---|
| ^{A}Unigene Cluster. ^{B}Bold indicates a transcript that was further tested by qRT -PCR. ^{C}*p* value obtained by ttest adjusted with Bonferroni's correction indicates a significant transcript variation in responders vsnon -responders. n.a.: not applicable. | | | | |

**Table 5. Performances of the number of selected transcripts for prediction of responsiveness.**

| **Number of selected transcripts** | **20** | **7** |
|---|---|---|
| Number of NR patients classified as NR^{B} | 8 | 8 |
| Number of NR patients classified as R^{B} | 2 | 2 |
| Number of R patients classified as R^{B} | 7 | 7 |
| Number of R patients classified as NR^{B} | 1 | 1 |
| Khi² test | 8.1 (p<0.01) | 8.1 (p<0.01) |
| Sensitivity^{B} | 80 % | 80 % |
| Specificity^{B} | 87.5 % | 87.5 % |
| Positive predictive value^{B} | 88.9 % | 88.9 % |
| Negative predictive value^{B} | 77.8 % | 77.8 % |

| | | |
|---|---|---|
| ^{B}by leave-one-out cross-validation with 20 patients including 10 non responders (NR) and 8 responders (R) (referred to as validation subsets 2 in text). | | |

### REFERENCES:

Throughout this application, various references describe the state of the art to which this invention pertains.
Allison, D.B., Cui, X., Page, G.P., and Sabripour, M. 2006. Microarray data analysis: from disarray to consolidation and consensus. Nat. Rev. Genet. 7:55-65.
Arnett, F.C., et al. 1988. The American Rheumatism Association 1987 revised criteria for the classification of rheumatoid arthritis. Arthritis. Rheum. 31:315-324.
Chen, L., et al. 2005. Hepatic gene expression discriminates responders and nonresponders in treatment of chronic hepatitis C viral infection. Gastroenterology. 128:1437-1444.
Coulouarn, C., et al. 2004. Altered gene expression in acute systemic inflammation detected by complete coverage of the human liver transcriptome. Hepatology. 39:353-364.
Drynda, S., et al. 2004. Proteome analysis reveals disease-associated marker proteins to differentiate RA patients from other inflammatory joint diseases with the potential to monitor anti-TNFalpha therapy. Pathol. Res. Prac. 200:165-171.
Fundel, K., Haag, J., Gebhard, P.M., Zimmer, R., and Aigner, T. 2008. Normalization strategies for mRNA expression data in cartilage research. Osteoarthritis. Cartilage.
Guillemin, F., Braincon, S., and Pourel, J. 1991. Measurement of the functional capacity in rheumatoid polyarthritis: a French adaptation of the Health Assessment Questionnaire (HAQ). Rev. Rhum. Mal. Osteoartic. 58:459-465.
Lequerré, T., et al. 2006. Gene profiling in white blood cells predicts infliximab responsiveness in rheumatoid arthritis. Arthritis. Res. Ther. 8:R105-115.
Lequerré, T., et al. 2007. Autoantibodies, metalloproteinases and bone markers in rheumatoid arthritis patients are unable to predict their responses to infliximab. Rheumatology. 46:446-453.
Olsen, N.J., Moore, J.H., and Aune, T.M. 2004. Gene expression signatures for autoimmune disease in peripheral blood mononuclear cells. Arthritis. Res. Ther. 6:120-128.
van Gestel, A.M., et al. 1996. Development and validation of the European League Against Rheumatism response criteria for rheumatoid arthritis. Comparison with the preliminary American College of Rheumatology and the World Health Organization/International League Against Rheumatism criteria. Arthritis. Rheum. 39:34-40.
Cohen, S., et al. 2002. Treatment of rheumatoid arthritis with anakinra, a recombinant human interleukin-1 receptor antagonist, in combination with methotrexate: results of a twenty-four-week, multicenter, randomized, double-blind, placebo-controlled trial. Arthritis. Rheum.46:614-624.

## Claims

1. A method for predicting the response of a patient affected with an IL-1 related disease selected from rheumatoid arthritis, spondylarthropathies, osteoarthritis, gouty arthritis, allergy, multiple sclerosis, autoimmune diabetes, autoimmune uveitis, and nephrotic syndrome to a treatment with anakinra, said method comprising a step of measuring the expression level of 7 genes in a biological sample of said patient, wherein said genes are GTF2F2, CCT3, CROT, HNRPA3, ARL15, TMED5, and NRG3.

2. The method according to claim 1, wherein said patient is affected with rheumatoid arthritis.

3. The method according to claim 2, wherein said patient is affected with rheumatoid arthritis that is active.

4. The method according to any of claims 1 to 3, which further comprises a step of measuring the expression level of 13 genes in said biological sample of said patient, wherein said genes are SERPINE1, MRPL40, EIF3S12, OVGP1, ZDHHC20, BAZ2B, F11R, SLC11A2, ONECUT1, MAP4, SLC15A4, CLEC2D, and RBM35B.

5. The method according to any of claims 1 to 4 which further comprises a step of comparing the combined expression level of said genes with reference values obtained from responder and non-responder group of patients.

6. The method according to any of claims 1 to 5 wherein the patient has received a treatment with methotrexate, azathropine or lefunomide.

7. The method according of any of claims 1 to 6 wherein said biological sample is blood.

8. The method according to any of claims 1 to 7 wherein the expression level is measured by quantifying the level of mRNa of said genes in the biological sample.

9. The method of claim 8 which comprises the steps of providing total RNAs extracted from PBMCs obtained from a blood sample of the patient, and subjecting the RNAs to amplication and hybridization to specific probes.

10. Use of anakinra for the preparation of a medicament for treating a patient with an IL-I related disease selected from rheumatoid arthritis, spondylarthropathies, osteoarthritis, gouty arthritis, allergy, multiple sclerosis, autoimmune diabetes, autoimmune uveitis, and nephrotic syndrome, wherein the patient has been classified as responder to a treatment with anakinra, by determining the expression level of 7 genes in a biological sample of said patient, wherein said genes are GTF2F2, CCT3, CROT, HNRPA3, ARL15, TMED5, and NRG3.

11. Use according to claim 10 of anakinra for the preparation of a medicament for treating a patient with rheumatoid arthritis, wherein the patient has been classified as responder to a treatment with anakinra, by determining the expression level of 7 genes in a biological sample of said patient, wherein said genes are GTF2F2, CCT3, CROT, HNRPA3, ARL15, TMED5, and NRG3.

## Patentansprüche

1. Ein Verfahren zur Vorhersage ob ein Patient, der von einer IL-1 gekoppelten Krankheit betroffen ist, ausgewählt von rheumatoider Arthritis, Spondylarthropathien, Arthrose, Gichtarthritis, Allergie, Multiple Sklerose, Autoimmundiabetes, Autoimmunuveitis und nephrotischem Syndrom, auf eine Behandlung mit Anakinra ansprechen würde, wobei das Verfahren einen Schritt der Bestimmung des Expressionsniveaus von 7 Genen einer biologischen Probe des Patienten umfasst, wobei diese Gene GTF2F2, CCT3, CROT, HNRPA3, ARL15, TMED5 und NRG3 sind.

2. Ein Verfahren nach Anspruch 1, wobei der Patient eine rheumatoide Arthritis hat.

3. Ein Verfahren nach Anspruch 2, wobei der Patient eine aktive rheumatoide Arthritis hat.

4. Das Verfahren nach einem der Ansprüche 1 bis 3, das weiter umfasst einen Schritt der Bestimmung des Expressionsniveaus von 13 Genen in der biologischen Probe des Patienten, wobei diese Gene SERPINE1, MRPL40, EIF3S12, OVGP1, ZDHHC20, BAZ2B, F11R, SLC11A2, ONECUT1, MAP4, SLC15A4, CLEC2D und RBM35B sind.

5. Das Verfahren nach einem der Ansprüche 1 bis 4, das weiter umfasst einen Schritt des Vergleichens des kombinierten Expressionsniveaus dieser Gene mit Referenzwerten, erhalten von Responder- und Nicht-Responderpatientengruppen.

6. Das Verfahren nach einem der Ansprüche 1 bis 5, wobei der Patient eine Behandlung mit Methotrexat, Azathioprin oder Lefunomid erhalten hat.

7. Das Verfahren nach einem der Ansprüche 1 bis 6, wobei die biologische Probe Blut ist.

8. Das Verfahren nach einem der Ansprüche 1 bis 7, wobei das Expressionsniveau durch die Quantifizierung des Niveaus der mRNA dieser Gene in der biologischen Probe bestimmt wird.

9. Das Verfahren nach Anspruch 8, welches die Schritte des Bereitstellens von Gesamt-RNAs extrahiert aus PBMCs erhalten aus einer Blutprobe des Patienten, und Unterwerfen der RNAs einer Amplifikation und Hybridisation zu spezifischen Proben umfasst.

10. Verwendung von Anakinra für die Herstellung eines Medikamentes zur Behandlung eines Patienten, der von einer IL-1 gekoppelten Krankheit betroffen ist, ausgewählt von rheumatoider Arthritis, Spondylarthropathie, Arthrose, Gichtarthritis, Allergie, Multiple Sklerose, Autoimmundiabetes, Autoimmunuveitis und nephrotischem Syndrom, wobei der Patient als Responder für eine Behandlung mit Anakinra durch die Bestimmung des Expressionsniveaus von 7 Genen in einer biologischen Probe des Patienten klassifiziert wurde, wobei diese Genen GTF2F2, CCT3, CROT, HNRPA3, ARL15, TMED5 und NRG3 sind.

11. Verwendung nach Anspruch 10 von Anakinra für die Herstellung eines Medikamentes zur Behandlung eines Patienten mit rheumatoider Arthritis, wobei der Patient als Responder für eine Behandlung mit Anakinra durch die Bestimmung des Expressionsniveaus von 7 Genen in einer biologischen Probe des Patienten klassifiziert wurde, wobei diese Genen GTF2F2, CCT3, CROT, HNRPA3, ARL15, TMED5 und NRG3 sind.

## Revendications

1. Procédé pour prédire la réponse à un traitement avec l'anakinra d'un patient ayant une maladie liée à IL-1, cette maladie étant une polyarthrite rhumatoïde, une spondylarthropathie, l'arthrose, l'arthrite gouteuse, une allergie, la sclérose en plaques, le diabète auto-immun, l'uvéite auto-immune ou le syndrome néphrotique, **caractérisé en ce que** ledit procédé comprend une étape de détermination du niveau d'expression de 7 gènes dans un échantillon biologique dudit patient, lesdits gènes étant GTF2F2, CCT3, CROT, HNRPA3, ARL15, TMED5 et NRG3.

2. Procédé selon la revendication 1, le patient ayant une polyarthrite rhumatoïde.

3. Procédé selon la revendication 2, le patient ayant une polyarthrite rhumatoïde active.

4. Procédé selon l'une quelconque des revendications 1 à 3, comprenant en outre une étape de détermination du niveau d'expression de 13 gènes dans l'échantillon biologique dudit patient, lesdits gènes étant SERPINE1, MRPL40, EIF3S12, OVGP1, ZDHHC20, BAZ2B, F11R, SLC11A2, ONECUT1, MAP4, SLC15A4, CLEC2D et RBM35B.

5. Procédé selon l'une quelconque des revendications 1 à 4, comprenant en outre une étape de comparaison du niveau d'expression combiné desdits gènes avec des valeurs de référence obtenues à partir des groupes de patients répondants ou non-répondants.

6. Procédé selon l'une quelconque des revendications 1 à 5, le patient ayant été traité avec du méthotrexate, de l'azathioprine ou du léflunomide.

7. Procédé selon l'une quelconque des revendications 1 à 6, l'échantillon biologique étant du sang.

8. Procédé selon l'une quelconque des revendications 1 à 7, le niveau d'expression étant déterminé par quantification du niveau d'ARNm desdits gènes dans l'échantillon biologique.

9. Procédé selon la revendication 8, comprenant les étapes de fourniture des ARN totaux extraits à partir des PBMC obtenues à partir d'un échantillon sanguin du patient, et de soumission des ARN à une amplification et à une hybridation à des sondes spécifiques.

10. Utilisation d'anakinra pour la préparation d'un médicament pour traiter un patient ayant une maladie liée à IL-1, cette maladie étant une polyarthrite rhumatoïde, une spondylarthropathie, l'arthrose, l'arthrite gouteuse, une allergie, la sclérose en plaques, le diabète auto-immun, l'uvéite auto-immune ou le syndrome néphrotique, le patient ayant été classé comme répondant au traitement avec l'anakinra par la détermination du niveau d'expression de 7 gènes dans un échantillon biologique dudit patient, lesdits gènes étant GTF2F2, CCT3, CROT, HNRPA3, ARL15, TMED5 et NRG3.

11. Utilisation selon la revendication 10 d'anakinra pour la préparation d'un médicament pour traiter un patient avec une polyarthrite rhumatoïde, le patient ayant été classé comme répondant au traitement avec l'anakinra par la détermination du niveau d'expression de 7 gènes dans un échantillon biologique dudit patient, lesdits gènes étant GTF2F2, CCT3, CROT, HNRPA3, ARL15, TMEDS et NRG3.
